# EUROPÄISCHE PATENTSCHRIFT

(11) **EP 0 589 302 B1**
(45) Veröffentlichungstag und Bekanntmachung des Hinweises auf die Patenterteilung: **04.02.1998**
(21) Anmeldenummer: 93114543.7
(22) Anmeldetag: 10.09.1993
(51) Int. Cl.: C07C 45/67, C07C 47/02

(54) **Verfahren zur Herstellung von Aldehyden**
Process for the preparation of aldehydes
Procédé pour la préparation d'aldéhydes

(30) Priorität: 21.09.1992 DE 4231490
(43) Veröffentlichungstag der Anmeldung: 30.03.1994
(73) Patentinhaber: BASF Aktiengesellschaft, 67063 Ludwigshafen (DE)
(72) Erfinder: Fischer, Rolf, Dr., D-6900 Heidelberg (DE)

(56) Entgegenhaltungen:
- EP-A- 0 055 797
- EP-A- 0 276 767
- WO-A-86/03486
- TETRAHEDRON LETTERS. Nr. 31 , 1976 , OXFORD GB Seiten 2707 - 2708 A.E. GREENE ET AL. 'Decarbalkoxylation of beta-Keto Esters - A new mild procedure'

## Beschreibung

Die vorliegende Erfindung betrifft ein Verfahren zur Herstellung von Aldehyden durch Umsetzung von geminalen Formylestern in Gegenwart von sauren Katalysatoren bei erhöhten Temperaturen.

Aus der DE-A-30 45 102 ist ein Verfahren zur Herstellung von substituierten Essigsäuren, deren Estern bzw. Acetonitrilen durch Umsetzung von Malon- oder Cyanessigestern bei erhöhten Temperaturen in Gegenwart von Katalysatoren bekannt.

Es ist aus Houben-Weyl, Methoden der organischen Chemie, vierte Auflage (1952), Band 8, Seiten 600 bis 612 bekannt, substituierte geminale Cyanessigester z.B. durch Alkylierung oder Acylierung von Cyanessigsäureestern herzustellen. Diese lassen sich nach saurer oder alkalischer Hydrolyse der Estergruppe durch Abspaltung der Carbonsäuregruppe in Nitrile überführen. Derartige Umsetzungen sind im allgemeinen mit Salzanfall verbunden.

Der vorliegenden Erfindung lag daher die Aufgabe zugrunde, den zuvor genannten Nachteilen abzuhelfen.

Demgemäß wurde ein neues und verbessertes Verfahren zur Herstellung von Aldehyden der allgemeinen Formel I 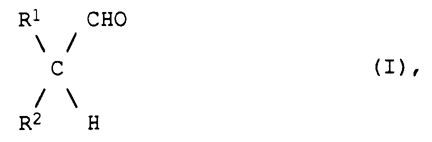 in der
- R¹, R²: Wasserstoff, C₁- bis C₁₂-Alkyl, C₃- bis C₈-Cycloalkyl, Acyl, Aryl oder C₇- bis C₂₀-Aralkyl oder gemeinsam - (CH₂)ₙ-X-(CH₂)ₘ-,
- X: Methylen, Sauerstoff, Schwefel, NH oder NR³ und
- n, m: 0 bis 8
bedeuten, gefunden, welches dadurch gekennzeichnet ist, daß man geminale Formylester der allgemeinen Formel II 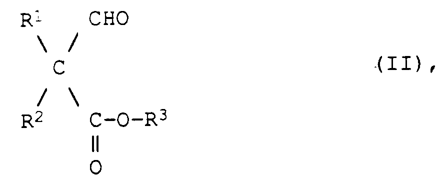 in der R¹ und R² die obengenannten Bedeutungen haben und R³ C₁-bis C₁₂-Alkyl bedeutet, in Gegenwart von sauren Katalysatoren bei Temperaturen von 150 bis 400°C umsetzt.

Das erfindungsgemäße Verfahren läßt sich wie folgt durchführen:

Die geminalen Formylester II werden bei erhöhten Temperaturen durch Umsetzung an sauren Katalysatoren in die Aldehyde I umgewandelt.

Die Umsetzung kann diskontinuierlich oder kontinuierlich bei Temperaturen von 150 bis 400°C, vorzugsweise von 170 bis 400°C, insbesondere von 200 bis 300°C durchgeführt werden. Der Druck bei der Reaktion ist unkritisch, vorteilhaft wählt man Drücke von 0,1 bis 100 bar, insbesondere von 1 bis 10 bar. Die Umsetzung der geminalen Formylester II zu Aldehyden I kann in der Flüssigphase oder bevorzugt in der Gasphase erfolgen. Man arbeitet dabei vorteilhaft mit einer Katalysatorbelastung von 0,1 bis 10 g, insbesondere 0,1 bis 5 g II pro g Katalysator und Stunde.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise Ether wie Diethylether, Tetrahydrofuran und Dioxan, Aromaten wie Benzol, Toluol und die Xylole, chlorierte Kohlenwasserstoffe wie Chloroform und Methylenchlorid, bevorzugt Alkohole wie beispielsweise C₁- bis C₈-Alkohole, insbesondere C₁-bis C₅-Alkohole wie Methanol, Ethanol, n-Propanol, i-Propanol, Butanole und Pentanole verwendet werden. Die Menge an Lösungsmittel beträgt in der Regel, bezogen auf eingesetztes II, 5 bis 90 Gew.-%.

Die Umsetzung kann diskontinuierlich oder kontinuierlich als Festbettreaktion mit Festbettkatalysatoren, beispielsweise in Sumpf- oder Rieselfahrweise in der Flüssigphase oder in der Gasphase, z.B. im Wirbelbett oder aber mit in der Flüssigphase suspendierten Festbettkatalysatoren durchgeführt werden.

Die Umsetzung der geminalen Formylester II in der Flüssigphase wird beispielsweise so durchgeführt, daß man ein Gemisch aus II und gegebenenfalls einem Lösungsmittel in Gegenwart eines suspendierten Festbettkatalysators auf die gewünschte Reaktionstemperatur erhitzt. Nach Ablauf der Reaktion wird das Reaktionsgemisch abgekühlt und der Katalysator, z.B. durch Filtration oder Neutralisation, entfernt.

Das Reaktionsgemisch kann anschließend zur Gewinnung der gewünschten Aldehyde I fraktionierend destilliert werden.

Nach einer bevorzugten Ausführungsform des erfindungsgemäßen Verfahrens in der Gasphase an Festbett- oder Wirbelbettkatalysatoren und in Gegenwart eines Alkohols kann z.B. folgendermaßen verfahren werden:

Ein Gemisch aus II und dem jeweiligen Alkohol wird verdampft und dann, gegebenenfalls zusammen mit einem Inertgas wie Stickstoff, Kohlendioxid oder Argon, bei der gewünschten Reaktionstemperatur gasförmig über einen fest angeordneten oder wirbelnden Katalysator geleitet, wobei der Wirbelbett-Katalysator in auf- und abwirbelnder Bewegung gehalten wird. Der Reaktionsaustrag wird mittels geeigneter Kühlvorrich- tung kondensiert und anschließend durch fraktionierende Destillation aufgearbeitet. Die gewünschte Verbindung I wird abgetrennt. Nicht umgesetzte Verbindung II kann gegebenenfalls in die Reaktion zurückgeführt werden.

Grundsätzlich sind verschiedenste saure Katalysatoren wie Mineralsäuren, Sulfonsäuren, Carbonsäuren und Lewissäuren geeignet. Als saure Katalysatoren eignen sich besonders Metalloxide der Elemente der ersten bis fünften Hauptgruppe und der ersten bis achten Nebengruppe sowie Oxide der Lanthaniden. Beispiele für derartige Metalloxide sind Bortrioxid, Aluminiumoxid, Siliziumdioxid, Titandioxid, Zinkoxid, Nioboxid, Vanadinpentoxid, Molybdänoxid, Ceroxid und Wolframoxid, bevorzugt Aluminiumoxid, Siliziumdioxid, Titandioxid, Zirkondioxid, Bortrioxid, Vanadinpentoxid, Molybdänoxid, Wolframoxid und deren Gemische. Besonders bevorzugt ist Aluminiumoxid.

Als saure Katalysatoren eignen sich ferner saure Phosphate, saure Ionenaustauscher, Silikate und Zeolithe wie beispielsweise Zeolithe der Mordenit-Gruppe, X-, Y- oder L-Zeolithe wie Mordenit, Erionit und Faujasit, bevorzugt sind Zeolithe mit Pentasilstruktur wie ZSM-5-, ZSM-11-und ZBM-10-Zeolithe, besonders bevorzugt ZSM-5- und ZSM-11-Zeolithe.

Als saure Katalysatoren eigenen sich weiterhin Heteropolysäuren wie H₃[PW₁₂O₄₀], H₃[PMo₁₂O₄₀] oder H₄[SiW₁₂O₄₀], bevorzugt H₃[PW₁₂O₄₀] und H₃[PMo₁₂O₄₀], besonders bevorzugt H₃[PW₁₂O₄₀].

Das Zwischenglied X, die Substituenten R¹, R², R³ und die Indizes n und m in den Verbindungen I und II haben folgende Bedeutungen:
- R¹, R²: - unabhängig voneinander
- Wasserstoff,
- C₁- bis C₁₂-Alkyl wie Methyl, Ethyl, n-Propyl, iso-Propyl, n-Butyl, iso-Butyl, sec. -Butyl, tert.-Butyl, n-Pentyl, iso-Pentyl, sec.-Pentyl, neo-Pentyl, 1,2-Di-methylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-He-xyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl wie Cyclopropyl, Cyclobutyl, Cyclopentyl, Cyclohexyl, Cycloheptyl und Cyclooctyl, be- vorzugt Cyclopentyl, Cyclohexyl und Cyclooctyl,
- Acyl wie Acetyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- -(CH₂)ₙ-X-(CH₂)ₘ-,
- X: - Methylen (-CH₂-),
- Sauerstoff,
- Schwefel,
- NH,
- NR³,
- R³: - C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl und
- n, m: - jeweils eine ganze Zahl von 0 bis 8 wie 0, 1, 2, 3, 4, 5, 6, 7 und 8, bevorzugt jeweils eine ganze Zahl von 1 bis 4 wie 1, 2, 3 und 4.

Geeignete geminale Formylester der Formel II sind z.B. 2-Formylvaleriansäuremethylester und 2-Formylpropionsäuremethylester.

Die für die erfindungsgemäße Umsetzung benötigten Verbindungen II sind allgemein bekannt.

Die nach dem erfindungsgemäßen Verfahren erhältlichen Aldehyde I stellen wertvolle, vielseitig für organische Synthese verwendbare Zwischenprodukte dar.

### Beispiele

Die Versuche wurden so durchgeführt, daß Lösungen der Ausgangsverbindungen II in Methanol bei unterschiedlichen Temperaturen kontinuierlich auf 5 g γ-Aluminiumoxid gepumpt wurden, das sich in einer Edelstahl-Reaktorwendel befand. Der Reaktor wurde in einem Heißluftofen auf die gewünschte Temperatur aufgeheizt. Dem Al₂O₃-Reaktorbett war eine Schicht aus Inertmaterial vorgelagert, in dem die zugepumpte Lösung verdampfte. Der gasförmige Austrag wurde kondensiert, gaschromatographisch analysiert und gegebenenfalls destillativ aufgearbeitet.

### Beispiel 1

59,6 g einer 20 %igen Lösung von 2-Formylvaleriansäuremethylester in Methanol wurden bei 200°C innerhalb von 7,5 Stunden dem Reaktor zugeführt. Der vereinigte Austrag wurde an einer Drehbandkolonne destilliert. In dem Destillat wurden 4,6 g n-Valeraldehyd festgestellt (65 %, bezogen auf eingesetzten 2-Formylvaleriansäuremethylester).

### Beispiel 2

42,5 g einer 20 %igen Lösung von 2-Formyl-2-methylpropionsäuremethylester in Methanol wurden innerhalb von 5 Stunden dem Reaktor bei 200°C zugeführt. Der vereinigte Austrag wurde gaschromatographisch analysiert (innerer Standard). Dabei wurde eine i-Butylraldehyd-Ausbeute von 29 % (bezogen auf eingesetzten Formylester) gefunden. Weiterhin wurden 44 % unumgesetzter Formylester festgestellt. Die i-Butyraldehyd-Selektivität beträgt demnach 52 %.

## Patentansprüche

1. Verfahren zur Herstellung von Aldehyden der allgemeinen Formel I 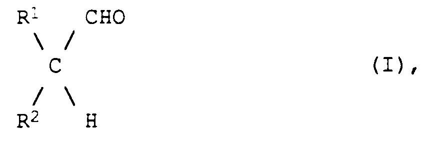 in der
R¹, R² Wasserstoff, C₁- bis C₁₂-Alkyl, C₃- bis C₈-Cycloalkyl, Acyl, Aryl oder C₇- bis C₂₀-Aralkyl oder gemeinsam -(CH₂)ₙ-X-(CH₂)ₘ-,
X Methylen, Sauerstoff, Schwefel, NH oder NR³ und
n, m 0 bis 8
bedeuten, dadurch gekennzeichnet, daß man geminale Formylester der allgemeinen Formel II 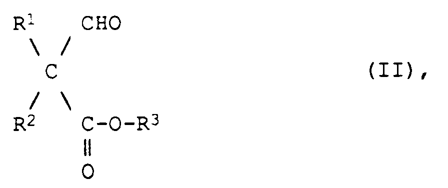 in der R¹ und R² die obengenannten Bedeutungen haben und R³ C₁- bis C₁₂-Alkyl bedeutet, in Gegenwart von sauren Katalysatoren bei Temperaturen von 150 bis 400°C umsetzt.

2. Verfahren zur Herstellung von Aldehyden I nach Anspruch 1, dadurch gekennzeichnet, daß man als saure Katalysatoren Metalloxide der Elemente der ersten bis fünften Hauptgruppe und/oder der ersten bis achten Nebengruppe und/oder der Lanthanidengruppe des Periodensystems der Elemente und/oder Zeolithe und/oder Heteropolysäuren verwendet.

3. Verfahren zur Herstellung von Aldehyden I nach Anspruch 1, dadurch gekennzeichnet, daß man als saure Katalysatoren Aluminiumoxid, Siliciumdioxid, Titandioxid, Zirkondioxid, Vanadinpentoxid und/oder Bortrioxid und/oder Oxide des Chroms, Molybdäns und/oder Wolframs verwendet.

4. Verfahren zur Herstellung von Aldehyden I nach Anspruch 1, dadurch gekennzeichnet, daß man als Katalysatoren Zeolithe oder Heteropolysäuren verwendet.

5. Verfahren zur Herstellung von Aldehyden I nach Anspruch 1, dadurch gekennzeichnet, daß man als Katalysator Aluminiumoxid verwendet.

6. Verfahren zur Herstellung von Aldehyden I nach Anspruch 1, dadurch gekennzeichnet, daß man in Gegenwart von Lösungsmitteln arbeitet.

7. Verfahren zur Herstellung von Aldehyden I nach den Ansprüchen 1 und 6, dadurch gekennzeichnet, daß man in Gegenwart von Alkoholen arbeitet.

8. Verfahren zur Herstellung von Aldehyden I nach den Ansprüchen 1 und 7, dadurch gekennzeichnet, daß man in der Gasphase arbeitet.

## Claims

1. A process for preparing aldehydes of the general formula I 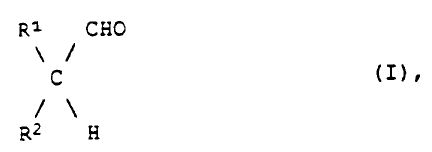 where
R¹, R² are hydrogen, C₁-C₁₂-alkyl, C₃-C₈-cycloalkyl, acyl, aryl or C₇-C₂₀-aralkyl or together are -(CH₂)ₙ-X-(CH₂)ₘ-,
X is methylene, oxygen, sulfur, NH or NR³ and
n, m are 0 to 8
which comprises reacting geminal formyl esters of the general formula II 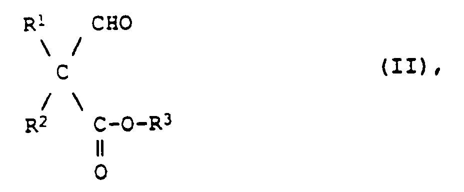 where R¹ and R² have the abovementioned meanings, and R³ is C₁-C₁₂-alkyl, in the presence of acidic catalysts at from 150 to 400°C.

2. A process for preparing aldehydes I as claimed in claim 1, wherein metal oxides of the elements of the first to fifth main group and/or of the first to eighth subgroup and/or of the lanthanide group of the periodic table of the elements and/or zeolites and/or heteropoly-acids are used as acidic catalysts.

3. A process for preparing aldehydes I as claimed in claim 1, wherein aluminum oxide, silicon dioxide, titanium dioxide, zirconium dioxide, vanadium pentoxide and/or boron trioxide and/or oxides of chromium, molybdenum and/or tungsten are used as acidic catalysts.

4. A process for preparing aldehydes I as claimed in claim 1, wherein zeolites or heteropolyacids are used as catalysts.

5. A process for preparing aldehydes I as claimed in claim 1, wherein aluminum oxide is used as catalyst.

6. A process for preparing aldehydes I as claimed in claim 1, which is carried out in the presence of solvents.

7. A process for preparing aldehydes I as claimed in either of claims 1 and 6, which is carried out in the presence of alcohols.

8. A process for preparing aldehydes I as claimed in either of claims 1 and 7, which is carried out in the gas phase.

## Revendications

1. Procedé de préparation d'aldéhydes de formule générale I 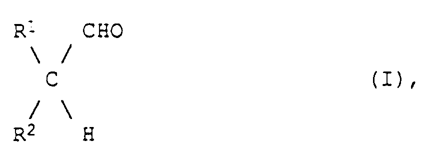 dans laquelle
R¹, R² représentent des atomes d'hydrogène ou des restes alkyle en C₁-C₁₂, cycloalkyle en C₃-C₈, acyle, aryle ou aralkyle en C₇-C₂₀ ou représentent ensemble -(CH₂)ₙ-X-(CH₂)ₘ-,
X représente un reste méthylène, un atome d'oxygène, de soufre ou un groupement NH ou NR³ et
n, m sont des nombres de 0 à 8,
caractérisé en ce que l'on fait réagir des esters formyliques géminés de formule générale II 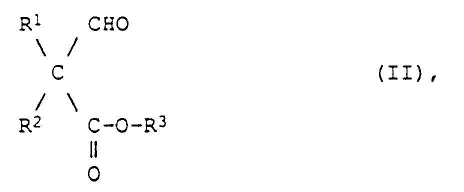 dans laquelle R¹ et R² ont les significations données ci-dessus et R³ représente un reste alkyle en C₁-C₁₂, en présence de catalyseurs acides à des températures de 150 à 400°C.

2. Procédé de préparation d'aldéhydes I selon la revendication 1, caractérisé en ce que l'on utilise, comme catalyseurs acides, des oxydes métalliques des éléments des groupes IA à VA et/ou des groupes IB à VIIB et VIII et/ou du groupe des lanthanides de la classification périodique des éléments et/ou des zéolithes et/ou des hétéropolyacides.

3. Procédé de préparation d'aldéhydes I selon la revendication 1, caractérisé en ce que l'on utilise, comme catalyseurs acides, de l'oxyde d'aluminium, du dioxyde de silicium, du dioxyde de titane, du dioxyde de zirconium, du pentoxyde de vanadium et/ou du trioxyde de bore et/ou des oxydes du chrome, du molybdène et/ou du tungstène.

4. Procédé de préparation d'aldéhydes I selon la revendication 1, caractérisé en ce que l'on utilise, comme catalyseurs, des zéolithes ou des hétéropolyacides.

5. Procédé de préparation d'aldéhydes I selon la revendication 1, caractérisé en ce que l'on utilise, comme catalyseur, de l'oxyde d'aluminium.

6. Procédé de préparation d'aldéhydes I selon la revendication 1, caractérisé en ce que l'on procède en présence de solvants.

7. Procédé de préparation d'aldéhydes I selon l'une quelconque des revendications 1 à 6, caractérisé en ce que l'on procède en présence d'alcools.

8. Procédé de préparation d'aldéhydes I selon l'une quelconque des revendications 1 a 7, caractérisé en ce que l'on procède en phase gazeuse.
